# EUROPEAN PATENT APPLICATION

(11) **EP 4 156 021 A1**
(43) Date of publication of application: **29.03.2023**
(21) Application number: 21199163.3
(22) Date of filing: 27.09.2021
(51) Int. Cl.: G06K 9/62, G06T 7/00, G16H 30/20

(54) **A METHOD AND SYSTEM FOR ANNOTATION OF MEDICAL IMAGES**

(71) Applicant: Smart Soft Healthcare, Varna (BG)
(72) Inventor: Georgiev, Nedelcho Todorov, Varna (BG)
(74) Representative: Zaboliene, Reda

(57) **Abstract**

The present invention relates to image data processing, in particular to a method and system for annotation of medical images. The method comprises: retrieving a plurality of medical images; preparing reports by physicians based on assessment of the retrieved medical images; sending the initial data that contains images and reports from database to merge selector, wherein the data is prepared and exported to annotation; checking the returned annotated data for discrepancies and disagreements; sending correctly annotated data without discrepancies and disagreements from the database for the input to the model training generating a trained model which makes automatic data annotations, wherein all decisions made by the trained model are checked additionally, and if there are discrepancies, the decisions are corrected and returned to the database for the improvement of the model training. The method further comprises that disagreements are solved by the arbiter, and data generated from an already trained model is merged with the data in the database to make it easier to annotate future data. The invention also relates to a data processing system comprising means for carrying out the steps of the method mentioned above.

## Description

### FIELD OF THE INVENTION

The present invention relates to image data processing, in particular to a method and system for annotation of medical images.

### BACKGROUND

Machine Learning is an application of Artificial Intelligence. It provides the systems the ability to automatically learn and improve from by training without being explicitly programmed.

Algorithms are trained to find patterns and features in data in order to make decisions and predictions based on new data.

There are three primary machine learning methods: supervised machine learning, unsupervised and semi-supervised learning.

Supervised machine learning trains itself on a labeled data set. The data is labeled with information that the machine learning model is being built to determine. This method requires less training data than other machine learning methods. It also makes training easier because the results of the model can be compared to actual labeled results.

Unsupervised machine learning ingests unlabeled data and uses algorithms to extract meaningful features needed to label, sort and classify the data in real time. This method is less about automating decisions and predictions, but more about identifying patterns and relationships in data.

Semi-supervised learning method, during training uses a smaller labeled data set to guide classification and feature extraction from a larger, unlabeled data set. Semi-supervised learning can solve the problem of having not enough labeled data (or not being able to afford to label enough data) to train a supervised learning algorithm.

The process of learning begins with observation of different examples, experience or instructions in order to make better decisions in the future, based on the given examples. The primary goal is the computer or system to learn automatically, without human intervention or assistance.

There are four basic steps for building a machine learning application.

The first step is to select and prepare the training data set.

The training data is a data set representative of the data the model will use to solve the problem for which it is designed. Sometimes the training data is labeled data - call out features and classifications the model will need to identify. Other data is unlabeled and the model will need to extract those features and assign classifications on its own.

The training data needs to be well prepared - randomized, de-duped, and checked for imbalances or biases that could impact the training. It should also be divided into two subsets: the training subset, which will be used to train the application, and the evaluation subset, used to test and refine it.

The second step is to choose a machine learning algorithm to run on the training data set.

The type of algorithm that will be used depends on the type (labeled or unlabeled), amount of data in the training data set and on the type of the problem that needs to be solved.

Some types of machine learning algorithms that use labeled data are: regression algorithms, decision trees and instance-based algorithms. For unlabeled data such algorithms are: clustering algorithms, association algorithms and neural networks.

The third step in machine learning is to train the algorithm to create the model. It involves running variables through the algorithm, comparing the output with the results it should have produced, adjusting weights and biases within the algorithm that might yield a more accurate result, and running the variables again until the algorithm returns the correct result most of the time. The accuracy is evaluated based on the performance of the model on the test set compared to the test set's ground truth (the test set contains pre-labeled data).

And the fourth and last step is to use and improve the model in order to be more effective with future variations.

In the context of clinical diagnosis, two classes of machine learning algorithms exist. These algorithmic techniques can be useful in extracting information or to model large data sets. The two classes are fully automated algorithms for diagnosis and algorithms that assist an expert clinician to perform the diagnosis, or computer assisted diagnosis. The purpose of computer-based systems is to support clinicians in their decision making.

According to Schick, F. 2016. Tissue segmentation: a crucial tool for quantitative MRI and visualization of anatomical structures, Magn Reason Mate Phy 29: 89-93, medical image segmentation is an important part for an assistant type of software. Segmentation of tissue types or organs is well established for X-ray-based computer tomography. Tissue segmentation aims at partitioning an image into segments corresponding to different tissue classes. These classes are biologically defined as specific types of tissue, whole organs, or sub-regions of organs. Areas with pathologies, such as tumors, herniation or inflammation are also relevant for segmentation. Medical image segmentation works well in two-dimensional or three-dimensional data sets with high contrast between the tissue classes to be separated.

Image segmentation is considered the most essential medical imaging process as it extracts the region of interest (ROI) through a semi-automatic or fully automated process. It divides an image into areas based on a specified description, such as segmenting body organs/tissues in the medical applications for border detection, tumor detection/segmentation, herniation detection/segmentation and mass detection.

For spine, Magnetic Resonance Imaging (MRI) is the modality of choice for intervertebral disc visualization and segmentation. MRI, for almost all spinal disorders, provides robust images of the spine with high quality soft-tissue visualization, much more detailed than results obtained with other modalities.

Spine is anatomically complex - 33 vertebrae, 23 intervertebral disks, spinal cord, branching nerve roots, connecting ribs, blood vessels, muscles, etc. An anatomically correct model, besides being complex and hard to create, would be computationally very demanding - possibly even unfeasible.

The most widely used classifications are topographic (by bodily region or system), anatomic (organ or tissue), physiological (function or tissue), pathological(by nature of the disease process), etiologic (causal), juristic (by speed of advent of death), epidemiological, and statistical. The classification is performed by specialists, classifying the presence of a pathology, its grade of development (if necessary), the severity and the complications it causes, or may cause. Medical classification is used for transformation of medical diagnoses or procedure descriptions into standardized statistical code in a process known as clinical coding. Procedure classifications list procedure code, which is used to capture interventional data.

Tomography is a radiologic technique for obtaining clear X-ray images of deep internal structures by focusing on a specific plane within the body. Structures that are obscured by overlying organs and soft tissues that are insufficiently delineated on conventional X-rays can thus be adequately visualized.

The term "computed tomography", or CT, refers to a computerized X-ray imaging procedure in which a narrow beam of X-rays is aimed at a patient and quickly rotated around the body, producing signals that are processed by the machine's computer to generate cross-sectional images (or "slices") of the body. These slices are called tomographic images and contain more detailed information than conventional X-rays. Once a number of successive slices are collected by the machine's computer, they can be digitally "stacked" together to form a three-dimensional image of the patient that allows for easier identification and location of basic structures as well as possible tumors or abnormalities.

Magnetic Resonance Imaging (MRI) is a non-invasive imaging technology that produces three dimensional detailed anatomical images. It is often used for disease detection, diagnosis, and treatment monitoring. It is based on sophisticated technology that excites and detects the change in the direction of the rotational axis of protons found in the water that makes up living tissues. MRIs employ powerful magnets which produce a strong magnetic field that forces protons in the body to align with that field. When a radiofrequency current is pulsed through the patient, the protons are stimulated, and spun out of equilibrium, straining against the pull of the magnetic field. When the radiofrequency field is turned off, the MRI sensors are able to detect the energy released as the protons realign with the magnetic field. The time it takes for the protons to realign with the magnetic field, as well as the amount of energy released, changes depending on the environment and the chemical nature of the molecules. To obtain an MRI image, a patient is placed inside a large magnet and must remain very still during the imaging process in order not to blur the image. Contrast agents (often containing the element Gadolinium) may be given to a patient intravenously before or during the MRI to increase the speed at which protons realign with the magnetic field. The faster the protons realign, the brighter the image.

According to Hanbury, A. 2008. A survey of methods for image annotation, Journal of Visual Languages and Computing 19: 617-627, datasets of annotated images are widely used as ground truth in object recognition and image annotation. The best method for creating ground truth is first to create a keyword vocabulary based on the requirements of the evaluation task and then to use this vocabulary in the manual annotation of images. The manual annotation is generally good due to world knowledge of the specialist (annotator). The only disadvantage is that it is time-consuming and labor-intensive. Also, a language barrier is possible between different annotators.

The manual annotation of data is performed by trained specialists. Their work is used as an input for the specific algorithm. Using a specialized program, specialists are given studies for classification and annotation. The studies include axial and sagittal MRI images on different slices of the lumbar spine. On slices that will be used for training the algorithm, they are able to segment the necessary tissues and pathologies. The process of this annotation includes four steps:

The first step is to find the mandatory tissues of the lumbar spine. The second step is to determine the severity of the pathologies directly associated with a tissue (e.g., herniation). The third step is segmenting (drawing over the MRI image) the relevant tissues. The fourth step is saving the already annotated study (classification and segmentation included) in a data base. Every tissue and pathology is being segmented in different color.

Annotating medical images is very useful. It includes techniques which allow an author to label, point to or indicate some feature of the image that is the focus of attention. It includes also textual commentary. Traditional methods for annotating images have allowed doctors to place pointers, textual information and labels. In this way they indicate structures contained in an image, but the information is static. Treatment planning or diagnosis for patients can be improved by comparing with other patient's clinical images with similar anatomical and pathological characteristics by understanding the image content. In order for this to happen, images should be annotated. Image annotation includes annotating images with labels. One way to annotate images is the doctor to types the annotation data in a word document stored along with the image in a database. The doctor or clinician writes reports to describe the image content of the respective image.

This annotation method is usually time consuming and can have many errors. This is because every medical specialist has different vocabulary and a way of describing the image content. Also because of language barriers the different specialists might be from different countries.

The idea of merging segmentations from the annotated data is not new and is presented by Yang, S. H.; Lee, U. S. 1997. Split-and-merge segmentation employing thresholding technique, Proceedings of International Conference on Image Processing, 26-29 Oct. 1997, but prior to the invention, it was done on non-overlapping segmentations. A thresholding technique is employed in the splitting phase of the split-and-merge segmentation scheme to reflect the image semantics to the image segmentation results directly.

According to Goswami, S. 2020. Reflection of Non-Maximum Supression (NMS), nNMS is a technique used in many computer vision algorithms. It is a class of algorithms to select one entity out of many overlapping entities. Most commonly, the criteria is some form of probability number along with some form of overlap measure. At the most basic level, most object detectors do some form of windowing. A form of windowing is using bounding boxes for the objects. Many, thousands of bounding boxes of various size and shapes are generated either directly on the image or on a feature of the image. These bounding boxes supposedly contain only one object, and a classifier is used to obtain a probability/score for each class. Once the detector outputs the large number of bounding boxes, it is necessary to pick the best ones. NMS is the most commonly used algorithm for this task. In essence, it is a form of clustering algorithm. In the invention all objects come from algorithms and humans, while the objects in the described NMS come from one algorithm that serves to evaluate overlapping.

It is different from the technique used in the present invention because it is based on the probability of objects according to an algorithm. In the invention, suppressions are done based on the correspondence between classifications and segmentation. Also, a check is performed to see if the source of segmentation is in agreement with the arbiter, and if it is the algorithm, the first examiner or the second examiner.

A "hard sample" is considered one where it is difficult for the machine learning model to correctly predict the labels of certain pathologies and tissues.

Hard sample mining is a method to distill a large amount of unlabeled data into a smaller high-quality labeled dataset. There are two methods of hard sample mining - offline and online. The offline method randomly selects a negative set to train a network, and after that, the trained model is used to select the hard negative pairs to retrain the fully connected layer of the network. The online method usually chooses hard samples in a mini-batch. It randomly samples images for a batch and selects the hardest positive and negative samples within the batch to form a triplet, which is called batch hard.

Another strategy to mine hard samples is a threshold. The combined mean of the positive pair distances and negative pair distances set are used to an adaptive threshold to mine the hard samples.

The difference in presented method is what is being done after the detection of a hard sample. Prior to the invention, it is used to increase its weight but that way in case it is an error which is actually deteriorating the training process. In present invention, it is transferred to a set that is ready for further data gathering.

Arbitration is a procedure in which both sides agree to let an impartial third side, the arbitrator, decide the case. The arbitrator may be a doctor, lawyer, or may be an expert in the field of the dispute, or in some cases, an arbitration panel. The arbitrator's decision, known as an award, is legally binding and can be enforced through the courts.

In adjudication, the decision is the responsibility of a third side adjudicator selected by the parties to the dispute. Adjudication decisions are binding unless and until they are revised by subsequent arbitration. Prior to the invention, adjudication is not used for segmentation or classification and segmentation.

According to Moučka, M. 2018. Semi-automatic tools for image segmentation. Master's thesis. Brno, semi- automatic segmentation combines the automatic segmentation technique, where image is segmented without physician interaction and the manual segmentation technique, where a physician manually marks regions in the image. SAS uses computer's ability to precisely delimit an object and combines it with the ability of a physician that understands the image on the high level and can recognize individual relevant objects and their positions. Effectiveness and efficiency of a SAS method depend on the proper combination of physicians' expertise and the capability of the computational method.

It differs from the present invention in the fact that there is no prefiltering process, and the full algorithm output is provided for corrections. The corrections are not checked for consistency with classification. Possible errors are not detected by using a second opinion.

The patent document US20160171682A1 (published on June 16,2016) covers the cloud-based image training and recognition. It receives a set of expert annotations of training images of a predetermined subject matter. The expert annotations include a clinical diagnosis for each image or region of interest in an image, training and testing one or more classification models. Each classification model yields a clinical diagnosis for each image and a confidence score for the diagnosis.

The difference compared with the present invention is that it is not cloud-based and provides not only classification, but segmentation as well. It also has filtration for the studies that will be given to physicians for annotation, as well as filtering of the studies themselves (which slices should be segmented with mandatory tissues and pathologies).

System and method for visual annotation and knowledge representation - US20080136838A1

The patent document US20080136838A1 (published on June 12, 2008) represents a method and system for visually annotating an image. The annotations and notes are stored in a structured vector representation alongside image information in a single, non-volatile and portable file or in a separate file from the image. The annotations are composed of point, line and polygon drawings and text symbols, labels or definitions and captions or descriptions. Also, the annotations can be retrieved for editing, printing, display and indexing and reporting.

The difference compared with the present invention is that it covers segmentation and classification information, while the above mentioned covers only the visualization of combined image and text information.

### SUMMARY OF THE INVENTION

A disclosure presents a method and a system for annotation of medical images. The presented method includes making of annotations, their checking and validation, data preparation for a model training and improvement of the model training.

In the disclosure, a term "segmentation" refers to a procedure of segmenting objects of interest in a medical image.

In the disclosure, a term "classification" refers to classifying medical images into different categories to help physicians in disease diagnosis or further research.

In the disclosure, a term "annotation" refers to a process of labeling the medical imaging data like Ultrasound, MRI, and CT Scan, etc. for machine learning training.

The method comprises:
retrieving a plurality of medical images;
preparing reports by physicians based on assessment of the retrieved medical images;
sending the initial data that contains images and reports from database to merge selector, wherein the data is prepared and exported to annotation;
checking the returned annotated data for discrepancies and disagreements;
sending correctly annotated data without discrepancies and disagreements from the database for the input to the model training generating a trained model which makes automatic data annotation;
wherein all decisions made by the trained model are checked additionally, and if there are discrepancies, the decisions are corrected and returned to the database for the improvement of the model training.

Medical images are provided by image acquisition apparatus or retrieved from the image database. The images and reports are anonymized to preserve identity of the patient.

When the data is prepared for classification and segmentations, all the slice of a study are checked. Only the necessary slices that need annotation, or where there is valuable information for the software to learn from, are sent. Such slices are chosen based on disagreements or missing important data. On these selected slices, annotations (segmentation and classification edits) are made based on a given structured report that lists pathologies and mandatory missing/incorrect tissues.

All possible segmentations from the initial annotation are used. Afterwards, an algorithm segments all mandatory tissues. Any tissues that are missing after the initial annotation are added by the algorithm without rewriting the already present ones. This is done using an "intelligent" Segmentation Merge that uses multiple outputs and compares them. The "intelligent" Segmentation Merge evaluates the outputs based on segmentation and classification consistency and reliability.

The method contains checks for correct segmentations. If a disagreement on the presence of a certain pathology occurs between the initial annotation and the algorithm, the algorithm's segmentation, if there is such, on that pathology is rejected. In this case the segmentation from the annotator is with greater weight. In case a tissue does not exist according to the initial annotation, the output of the algorithm for that tissue is also rejected.

The merge selector selects data with incorrect and/or mandatory segmentations, and incorrect classifications based on those segmentations. The chosen data is sent for a second check and annotation. This improves the quality of work and working time, because only a selected number of data are sent for correction.

In case there is a discrepancy/disagreement between the first annotation and the second annotation, the data is picked out and sent to an arbiter who makes the final decision, creating a majority opinion.

The method includes a handle for overlapping segmentations when segmentations are generated from the algorithm. Every tissue has a priority and if a tissue with lower priority overlaps another one, that part of it is deleted.

The data is considered "complete" and ready to be imported into the final database for training when:
- there are no disagreements left on the classifications;
- all the mandatory tissues are presented;
- all tissues for pathologies associated with the classifications are presented.

The database is used to train a model that uses the annotated images as input data. The trained model is the machine learning model (algorithm), making automated data annotation, and is the product of the successful model training. The data generated from the trained model is merged with the data in the database to make it easier to annotate the future data. The trained model can be used in the software as the main resource that makes the analytics. The presented method improves the learning quality of the model.

### BRIEF DESCRIPTION OF THE DRAWINGS

The following embodiments of the method for annotating medical images are described with reference to the enclosed figures:
Fig. 1 is a flow chart illustrating the overall structure of a system for annotation of medical images and the steps carried out for the method implementation.
Fig. 2 is a flow chart illustrating the inside structure of the method for annotation of medical images.

### DESCRIPTION OF THE PREFERRED EMBODIMENTS

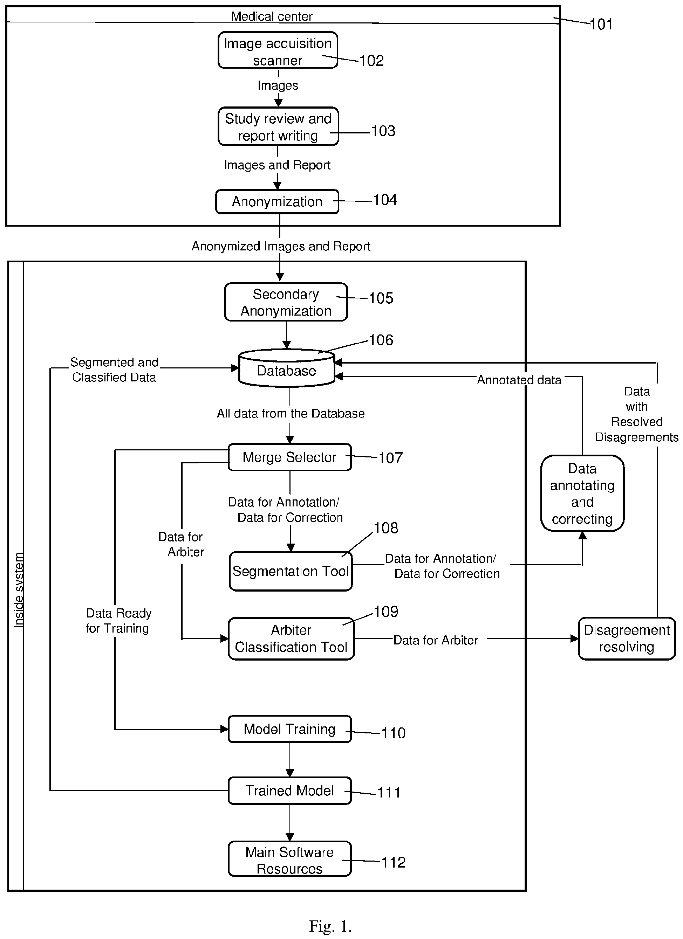

The present invention discloses a method and a system for annotation of medical images. Fig. 1 shows the overall structure of a system for annotation of medical images and the steps carried out for the method implementation.

At first, a plurality of medical images are acquired. The image can be provided by any kind of image acquisition apparatus (102), for example, X-ray apparatus, a computer tomography or a magnetic resonance scanning apparatus, that is located in a medical center (101), or can be retrieved from the image database.

Once the images are acquired, they are reviewed and analysed by a physician, and a report is issued (103) based on assessment of images.

Before the images and the report are sent outside the medical center (101), they are anonymized (104) to preserve the identity of the patient.

The studies (the data) containing images and reports goes through a second anonymization process to ensure their anonymity. Afterwards, the studies are saved in a database (106), and flagged as original (not yet annotated).

The studies are sent from the database (106) to the merge selector (107), wherein the said merge selector (107) selects which studies should be sent for annotation based on the available information for the certain study.

A specially designed segmentation tool (108) is used for data annotation. It is used to create the mandatory tissue and pathology segmentation. It is also additionally used to create classifications. Once the annotation process is complete, the studies are sent back and saved in the database (106). They are flagged as annotated during importing. The annotated studies are passed through the merge selector (108) once again, and if there are missing mandatory tissues, the segmentations from the algorithm are added. If still there are missing tissues or segmentation-based disagreements, in such cases only the slices in need of correction are selected and sent for a second check and annotation. Additionally, a structured report containing a list of the pathologies and mandatory tissues is included. The report is used to assist in determination of what corrections need to be made. The correction process follows the aforementioned method of annotation.

If there are classification disagreements, the studies are selected and sent to an arbiter. Using another specially designed tool (109), the arbiter resolves the disagreements by creating a majority opinion. The studies are then imported back into the database (106). After this step, they are passed through the merge selector (107) again.

The prepared and corrected studies are merged again. If all the mandatory tissues are presented and there are no more disagreements on any of the classifications, the studies are flagged as "ready for training". The "ready for training" studies are compiled and sent to be used as an input for the model training (110).

A study is considered "complete" and ready to be imported into the final database for training when:
- there are no disagreements left on the classifications;
- all the mandatory tissues are presented;
- all tissues for pathologies associated with the classifications are presented.

The model training (110) is the process of teaching a model how to make segmentations, and afterwards measurements based on them. Using these segmentations and measurements, the software can then make its own classification. Part of the studies are used during the learning process of the software. After training, the model is tested on a test set of studies, which are another part of already annotated data. In order to improve the efficiency of the model in relation to the volume of training data, the task is divided into subtasks, as they are trained simultaneously. Training is the minimization of a common goal function, which is a combination of the goal functions of each of the sub-tasks. The subtasks are localization, segmentation and classification of individual objects.

The information given by the software is compared to the ground truth, wherein the ground truth is the information from the annotation. Statistics are created based on this comparison to measure the accuracy of the software. If the accuracy is low, the model is trained again using an improved dataset as input. The model also uses some rule-based techniques to determine the presence, or specific characteristics of different pathologies.

The trained model (111) is the machine learning model (algorithm), which is the product of the successful model training (110). The trained model can then be used in a software as the main resource (112) that makes the analytics.

Additionally, the trained model is also can be used to create the aforementioned segmentations used in the merge selector when merging data before exporting it. This ensures that any missed tissues are added to the annotated data before the data is sent for review and/or correction.

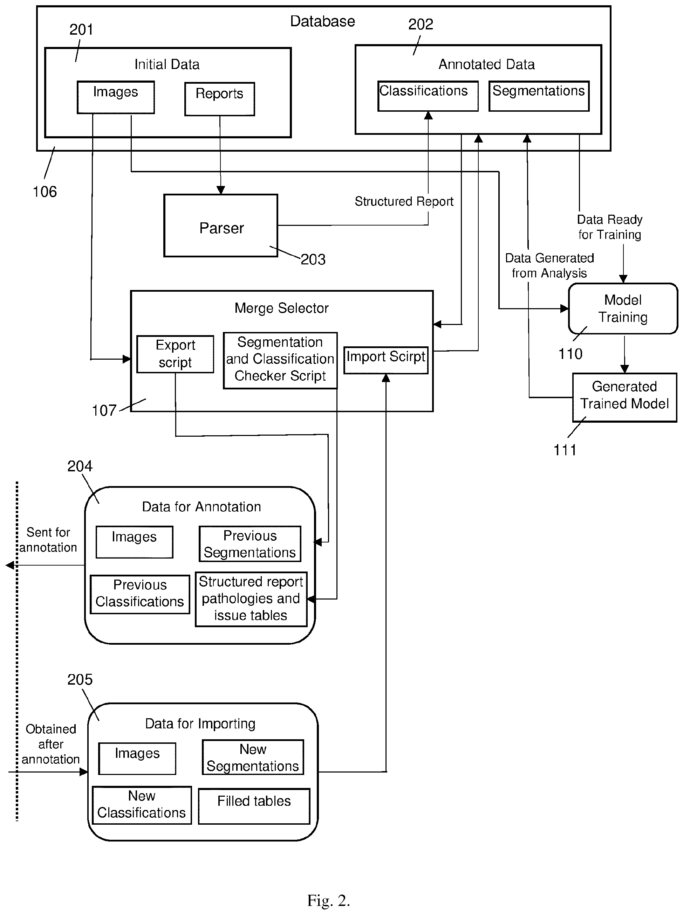

Fig. 2 is a flow chart illustrating the inside structure of the method for annotation of medical images.

The database (106) consists of initial data (201) containing images and reports from physicians, and annotated data (202) containing classifications and segmentations made for the studies.

The reports are put through a custom parser (203) that picks out the different pathologies and their specifications, and creates the initial classifications in the form of a structured report. The parser is a custom script that reads the provided report and extracts only the needed information through a report parsing process.

The initial data (201) goes through the merge selector (107), before being sent for initial annotation. Inside the merge selector (107) the data is prepared and exported. Then it is sent for annotation.

After the annotation process, the data is returned and is ready to be checked by the merge selector (107) again. Inside the merge selector (107), the data is firstly imported using an import script. The imported data (205) is merged with the data generated by the trained model (111). Afterwards, the data is checked by the merge selector (107) for any incorrect/missing segmentations and classification disagreements.

If there are incorrect/missing segmentations or classification disagreements between the initial annotation and the algorithm-generated one, the information is prepared again and is sent for further correction.

If all the mandatory tissues are presented, and are correctly segmented, the data is flagged as "ready for training". The data is then sent to be used as input for the model training (110) which implementation is presented above.

The model is the product of the whole training process. It is then used to generate data that is used by the merge selector (107) in the aforementioned steps.

In order to illustrate and describe the present invention, the above is a description of the most preferred embodiments. This is not an exhaustive or limiting description intended to determine the specific form or embodiment example. Obviously, many modifications and variations will be apparent to those skilled in the art. An embodiment is selected and described for those skilled in the art to better understand the principles of the present invention and their best practices for various embodiments with different modifications suitable for a particular use or application of the embodiment. It is intended that the scope of the invention be defined by the accompanying claim and its equivalents, in which all of the above terms have their broadest meaning unless otherwise indicated.

The embodiments described by those skilled in the art may be subject to modifications within the limits of the scope of the present invention as defined in the claim below.

## Claims

1. A method for improvement a training model (110) for annotation of medical images comprising:
retrieving a plurality of medical images;
preparing reports by physicians based on assessment of the retrieved medical images;
sending the initial data that contains images and reports from database (106) to merge selector (107), wherein the data is prepared and exported to annotation;
checking the returned annotated data for discrepancies and disagreements;
sending correctly annotated data without discrepancies and disagreements from the database for the input to the model training (110) generating a trained model (111) which makes automatic data annotation;
**characterized in that**
all decisions made by the trained model (111) are checked additionally, and if there are discrepancies, the decisions are corrected and returned to the database (106) for the improvement of the model training (110).

2. The method of claim 1, **characterized in that** the segmentation tool (108) is used for data annotation creating the mandatory tissue and pathology segmentation and classifications.

3. The method of claim 1, **characterized in that** data annotation is performed for data samples where the software can learn some new valuable information.

4. The method of claim 1, **characterized in that** the merge selector (107) selects data with incorrect and/or mandatory segmentations, and incorrect classifications based on those segmentations, and only the chosen data is sent for a second check and annotation.

5. The method of claim 1, **characterized in that** data for annotation is sent to an arbiter, if there are classification disagreements, wherein the arbiter makes the final decision creating a majority opinion.

6. The method of claim 1, **characterized in that** an intelligent segmentation merge process is used to take annotations from multiple inputs, compared by provided classification and segmentation.

7. The method of claim 1, **characterized in that** the trained model (111) creates automated annotations, wherein annotations are used by the intelligent segmentation merge process to fill in missing data.

8. The method of claim 1, **characterized in that** the data generated from the trained model (111) is merged with the data in the database (106) to make it easier to annotate the future data.

9. A data processing system comprising means for carrying out the steps of the method according to anyone of the claims 1 to 8.
